**Europäisches Patentamt**

**European Patent Office**

**Office européen des brevets**

(19)

(11) Publication number : **0 500 353 A1**

# (12) EUROPEAN PATENT APPLICATION

(21) Application number : **92301377.5**

(22) Date of filing : **19.02.92**

(51) Int. Cl.⁵ : **A61B 17/12**

(30) Priority : **20.02.91 US 658113**

(43) Date of publication of application :
**26.08.92 Bulletin 92/35**

(84) Designated Contracting States :
**AT BE CH DE DK ES FR GB GR IT LI LU NL SE**

(71) Applicant : **EDWARD WECK INC.**
**Weck Drive P.O. Box 12600**
**Research Triangle Park North Carolina 27709**
**(US)**

(72) Inventor : **Phillips, John C.**
**4520 Briarglen Lane**
**Holly Springs, North Carolina 27540 (US)**

(74) Representative : **Thomas, Roger Tamlyn**
**D. Young & Co. 10 Staple Inn**
**London WC1V 7RD (GB)**

(54) Hemostatic clip applier.

(57) A hemostatic clip applier is provided with a device for aligning the legs of hemostatic clips (22) with the crimping jaws (20,21) of the applier. The device enhances the operation of hemostatic clip appliers with clips having a "square" profile, i.e. clips having a width approximately equal to their lenght. The alignment device (40) interacts with portions of the clip on either side of the apex in order to rotate a skewed clip back into alignment with the jaws.

FIG. 2.

FIG. 3.

EP 0 500 353 A1

## BACKGROUND OF THE INVENTION

### FIELD OF THE INVENTION

The invention relates to hemostatic clip appliers. In particular, the invention provides an improvement in the manner in which a hemostatic clip is fed from a storage means within the applier to a working position between the crimping jaws of the applier.

### DESCRIPTION OF THE PRIOR ART

Automatic hemostatic clip appliers are well known in the prior art. Contemporary clip appliers are usually preloaded with a certain number of clips and are sterile and disposable. One such applier is shown in U.S. Patent 4,712,549 (Peters et al.) assigned to the assignee hereof and incorporated by reference herein.

All automatic clip appliers generally have several features in common. They all contain some storage means for storing a plurality of hemostatic clips, a dispensing means for removing the clips from the storage means, a jaw means for retaining and crimping a clip, some feed means for transferring a dispensed clip from the storage means to the jaw means and some actuating means for operating the feed means, jaw means and dispensing means in a sequential manner in order to crimp (usually) one clip at a time about a blood vessel to be ligated.

Other types of automatic hemostatic clip appliers are shown in U.S. Patent 3,777,538 (Weatherly et al.) and U.S. Patent 4,646,740 (Peters et al.), both assigned to the assignee hereof. Both of these appliers include the general features summarized above although the operation of these devices is somewhat different than the device shown in the '549 patent.

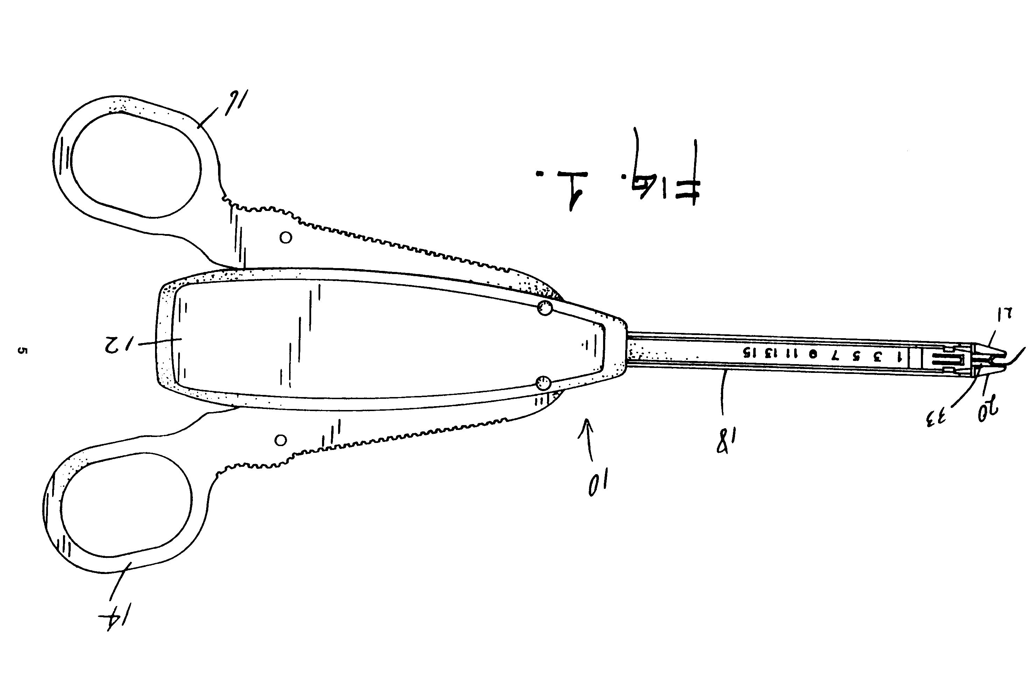

It has been found that the type of clip applier shown in the '549 patent has some desirable features which are often incorporated into many conventional appliers. For example, the ring handle actuating levers are situated at one end of the elongated applier and the crimping jaws are situated at the other end with the clips aligned in serial column fashion in a narrow and flat channel between the levers and the jaws. The legs of each clip push on the back of the preceding clip as the entire column of clips is moved by a pusher bar through the clip storage cartridge to the jaws. This type of arrangement of parts facilitates the use of the applier and is generally incorporated in the applier shown herein in Figure 1 which incorporates the subject invention.

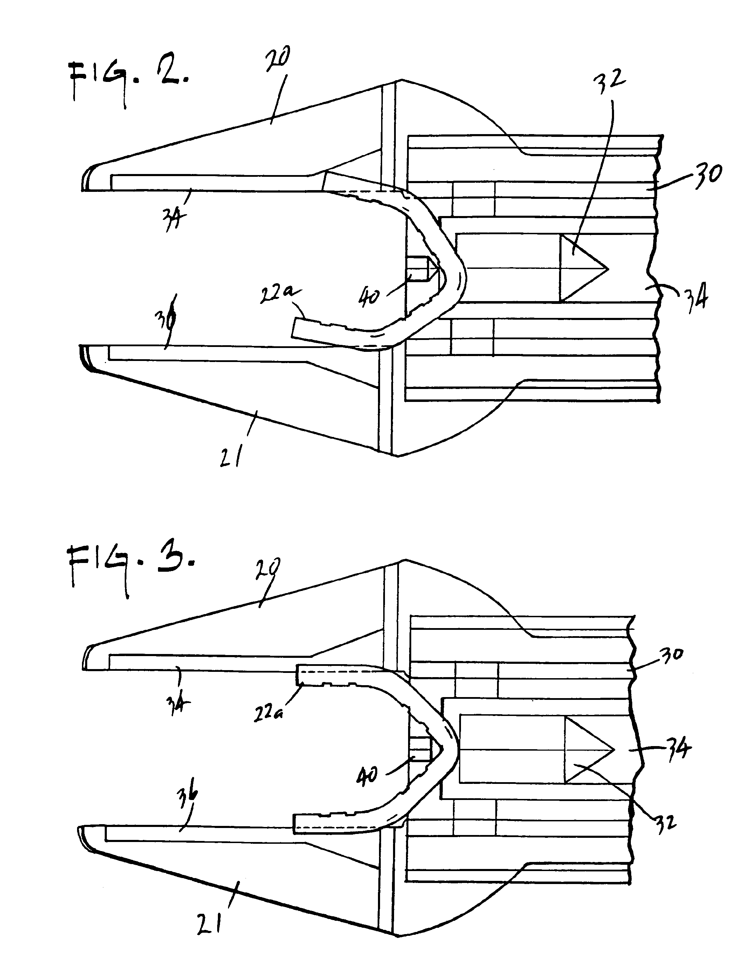

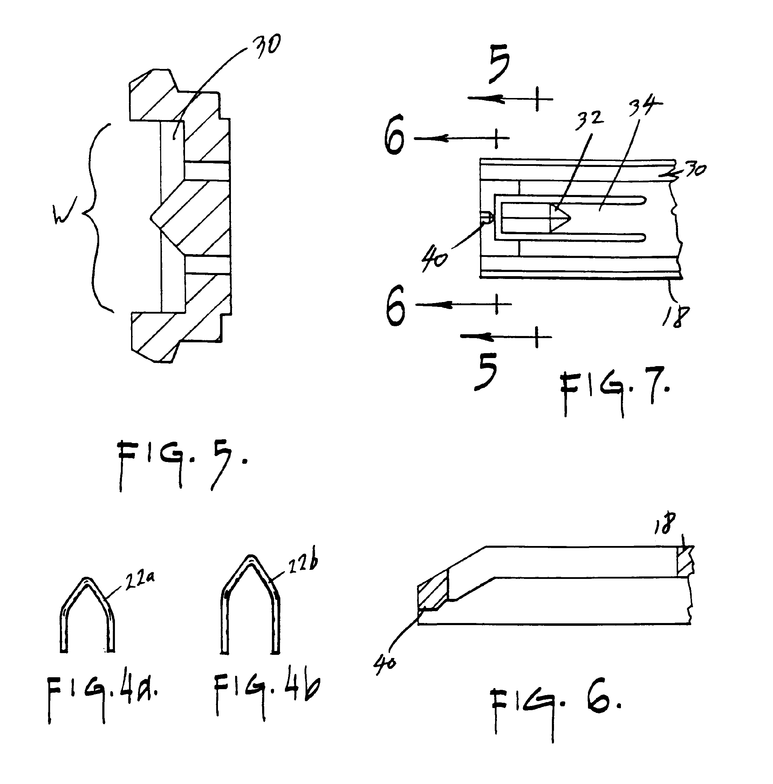

Hemostatic clips are generally integrally formed metallic or plastic U-shaped or C-shaped structures having a pair of generally parallel legs joined at an apex. When the clips are made of wire their legs are parallel and the clips retain their crimped position after crimping merely because of the inherent strength of the wire. When the clips are formed of plastic they are provided with other structures (e.g. hooks) to keep them closed. Clip appliers are generally manufactured in one common configuration which is modified to accommodate different numbers of clips and clips of different sizes depending upon the intended application. For purposes of describing the invention, the term "width" of a hemostatic clip will be hereinafter used to mean the distance between the parallel legs of the clip prior to its being crimped and the term "length" of the clip will be used to mean the distance between the tips of the legs and the apex. The width of the storage channel in the clip storage cartridge is approximately equal to the width of the clips to prevent clip rotation and assure that the clips are aligned with their open ends pointing distally. Depending upon the intended application, some hemostatic clips may have a width considerably less than their length (e.g. Figure 4b) and when such clips are situated in a channel within automatic hemostatic clip appliers such as those shown in the aforementioned patents, the clips are able to be pushed within the channel by a conventional pusher bar without any concern that the clips may rotate or become skewed within the channel. That is, the width of the clips is substantially equal to the width of the channel within which they are stored and pushed and, since the length of the clip is greater than the width of the channel there is no way for the clips to rotate out of alignment.

In certain size clips where the width is substantially equal to the length of the clips (e.g. Figure 4a) and there is a possibility that, because the width of the channel is approximately equal to the length of each clip the clips may rotate out of alignment and may even rotate a full 180° so the clip legs are pointing backwards (i.e. proximally rather than distally). If such clips become skewed this generally occurs at the transition from the channel to the jaws.

It was accordingly an aim of this invention to produce an improved hemostatic clip applier capable of assuring that clips fed to the jaws are properly aligned regardless of the size of the clips.

It was a further aim of this invention to produce an improvement in hemostatic clip appliers which enhances the operation of the appliers with relatively "square" clips while not adversely affecting operation of the applier with other clips.

### SUMMARY OF THE INVENTION

It has now been found that these aims can be achieved by the preferred embodiment of the invention which is an improvement in a clip applier for applying hemostatic clips, the applier comprising a storage means for storing a plurality of hemostatic clips, the storage means including a channel through which clips are slidably movable. The applier also has a crimping means for crimping a hemostatic clip, the crimping means including a pair of opposed jaws movable toward each other to crimp a hemostatic clip situated between the jaws, and a feed means for feeding a hemostatic clip to the crimping means. The improvement comprises a centering protrusion, extending on the longitudinal axis a predetermined distance into the path of the clips at a point immediately prior to the jaws, the protrusion being designed to contact each clip as it is advanced into position between the jaws and to cause a skewed clip to rotate about said centering protrusion into an aligned posture with the jaws. The centering protrusion is sufficiently resilient to be deflectable from its unbiased position to allow the clips to be moved past the centering protrusion into position between said jaws.

The centering protrusion is preferably tapered to a point of predetermined radius of curvature, said point facing away from the jaws.

## BRIEF DESCRIPTION OF THE DRAWINGS

Figure 1 is a plan view of an improved automatic hemostatic clip applier embodying the preferred embodiment of the invention.

Figure 2 is an exploded diagrammatic view of the jaws of the clip applier of Figure 1 showing a hemostatic clip in one possibled skewed orientation between the jaws of the applier.

Figure 3 is a view of Figure 2 showing the hemostatic clip in a properly oriented position.

Figures 4a and 4b are diagrammatic views of two different sizes of hemostatic clips.

Figure 5 is a cross-sectional view of a portion of the distal end of the storage channel of the clip applier of Figure 1, best seen in Figure 7, the cross-sectional view being taken along the lines 5-5.

Figure 6 is a cross-sectional view of a portion of the distal end of the storage channel of the clip applier of Figure 1, best seen in Figure 7, the cross-sectional view being taken along the lines 6-6.

Figure 7 is a diagrammatic bottom plan view of the distal end of the feed channel of the applier shown in Figure 1.

## DESCRIPTION OF THE PREFERRED EMBODIMENT

The preferred embodiment of the invention is included within automatic hemostatic clip applier 10 having a main body housing 12 and a pair of pivotable ring handles 14 and 16 graspable by the user at the proximal side of applier 10. Extending from the distal side of housing 12 along the longitudinal axis of applier 10 is a preloaded clip cartridge 18. At the distal end of clip cartridge 18 are a pair of jaws 20 and 21 between which is inserted a clip 22. It will be understood that longitudinally extending components such as feeder bar, jaw support means, etc. extend distally from housing 12 adjacent clip cartridge 18 in a fairly conventional manner.

The hemostatic clips within clip cartridge 18 are aligned within a channel 30, the width "w" of which is substantially equal to the width of the clips. The clips are arranged in a serial column fashion with their open ends facing distally such that the tips of the legs of one clip contact the rear of the preceding clip. The clips are pushed within the channel by a pusher bar (not shown).

Clips 22 may be relatively "square" as shown in Figure 4a with the width of each clip being substantially equal to its length or they may be relatively elongated as shown in Figure 4b with the width of the clip being substantially less than its length. The subject invention is intended to improve the operation of clip appliers with the "square" clips shown in Figure 4a although the invention will not adversely affect the operation of any clip applier with clips such as those shown in Figure 4b.

As best seen in Figure 2, because the width w of channel 30 is approximately equal to the width and length of clips 22a, there may be situations where the clip becomes skewed either within channel 30 or at the transition between channel 30 and jaws 20, 21.

The clip feeding mechanism does not form a part of the subject invention although it is described in aforementioned U.S. Patent 4,712,549 (Peters et al) which is incorporated by reference herein. Suffice it to say that the clips are stored in a serial fashion within one plane (i.e. in channel 30) and as they are pushed forward by the pusher bar each clip comes into contact with an integrally formed leaf spring 34 having a projection 32 extending downwardly from the extension of leaf spring 34 into the path of the clips 22. As the clips are urged distally by the pusher bar, the distalmost clip comes into contact with projection 32 which is tapered (pointing proximally as best seen in Figure 7) and urged out of alignment from channel 30 into a feed position proximally of jaws 20, 21. In the feed position the clip will be contacted by a feeder bar 33 (best seen in Figure 1) and pushed into grooves 34, 36 of jaws 20, 21. At this transition point because the groove 34, 36 may be flared proximally as best seen in Figures 2 and 3, the clips may become skewed and further pressure by the feeder bar may tend to jam the applier. This possibility is overcome by a centering alignment protrusion 40 extending downwardly from the distal tip of clip cartridge 18 a sufficient distance into the path of the clips (and on the longitudinal axis of the applier). Because of the tapered nature of the shoulders of the clip 22a on either side of its apex, if the clip is skewed when it contacts protrusion 40 it will tend to rotate about protrusion 40 until the latter comes into contact with the inside part of the apex of the clip as best seen in Figure 3 at which point the clip legs will be parallel to the grooves 34, 36 of jaws 20, 21 and further urging of the clip by the feeder bar 33 will force the clip over the resilient protrusion 40 into the grooves in preparation for use in a normal manner.

It will be understood by those skilled in the art that numerous modifications and improvements may be made to the preferred embodiment of the invention disclosed herein without departing from the spirit and scope hereof.

## Claims

1. An apparatus for applying C-shaped hemostatic clips each having a pair of substantially parallel legs joined by a central apex portion, the apparatus comprising:

   storage means for storing a plurality of said hemostatic clips, said storage means comprising a channel in which said clips are serially aligned and through which said clips are slidably movable;

   crimping means for crimping said hemostatic clips, said crimping means comprising a pair of opposed jaws movable toward each other to crimp one of said hemostatic clips situated between said jaws,

   feed means for sequentially urging said hemostatic clips along an axially aligned predetermined path to said crimping means;

   characterised by:

   a centering protrusion, extending a predetermined distance into said predetermined path on the longitudinal axis thereof at a point immediately prior to said jaws, for contacting each clip as it is advanced into position between said jaws and, if said contacted clip is skewed such that its legs are not aligned with said jaws, causing said contacted clip to rotate about said centering protrusion into an aligned posture with said jaws, said centering protrusion being sufficiently resilient to allow said contacted clip to be moved past said centering protrusion into position between said jaws.

2. An apparatus according to claim 1 wherein said centering protrusion is tapered to a point of predetermined radius of curvature, said point facing away from said jaws.

3. An apparatus according to claim 1 or 2 wherein the length of each said clip is substantially equal to its width.

Fig. 1.

FIG. 2.

FIG. 3.

FIG. 5.

FIG. 7.

FIG. 4a.

FIG. 4b.

FIG. 6.

**European Patent Office**

## EUROPEAN SEARCH REPORT

Application Number

EP    92 30 1377

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 409 569 (U.S. SURG. CORP.)<br>* column 19, line 46 - line 47; figures 2,5 *<br>--- | 1 | A61B17/12 |
| A | GB-A-2 128 477 (SENCO)<br>* page 6, line 100 - line 105; figures 6-8 *<br>--- | 1,2 | |
| A | WO-A-8 302 887 (U.S. SURG. CORP.)<br>* page 15, line 24 - line 27; figure 17 *<br>--- | 1 | |
| A | US-A-4 821 721 (CHIN ET AL.)<br>* column 3, line 54 - line 60; figure 6 *<br>----- | 1 | |
| | | | TECHNICAL FIELDS SEARCHED (Int. Cl.5)<br><br>A61B |

The present search report has been drawn up for all claims

| Place of search | Date of completion of the search | Examiner |
|---|---|---|
| THE HAGUE | 26 MAY 1992 | MOERS R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)